# EUROPEAN PATENT APPLICATION

(11) **EP 4 597 077 A1**
(43) Date of publication of application: **06.08.2025**
(21) Application number: 25152840.2
(22) Date of filing: 20.01.2025
(51) Int. Cl.: G01N 21/65, G01N 21/85, G01N 33/22

(54) **INSTALLATION AND METHOD FOR THE ANALYSIS OF GAS IN A NETWORK**

(30) Priority: 05.02.2024 IT 202400002328
(71) Applicant: Pietro Fiorentini S.p.A., 36057 Arcugnano (VI) (IT)
(72) Inventor: NARDI, Mario Pietro, 36057 Arcugnano (VI) (IT)
(74) Representative: Braidotti, Andrea

(57) **Abstract**

Installation (1) for analysing a gas flow circulating in a network (10) and/or between networks, said network (10) and/or networks comprising a main circuit (11) for the passage of the gas with a first point (12) and a second point (13) connected to each other by a main line (14) for the passage of the gas, said installation (1) being characterised in that it comprises an analysis circuit (20) with a further line (21) for the passage of the gas to be analysed, and wherein said further line (21):
- at the inlet and upstream is fluidically connected or connectable with the first point (12), to thus collect the gas to be analysed from the main circuit (11), and
- at the outlet and downstream it is fluidically connected or connectable with the second point (13), to thus reintroduce the gas into the main circuit (11),
and characterised in that the analysis circuit (20) of the installation (1) also comprises an analyser device (30), preferably of the optical type, which is mounted on the further line (21) of the analysis circuit (20) between the first point (12) and the second point (13) and which is configured to analyse the gas that passes through the further line (21) of the analysis circuit (20).

## Description

### TECHNICAL FIELD

The present invention concerns an installation for the analysis of gas in a network, in particular to determine the composition and/or quality of the gas circulating in a network and/or between networks.

The present invention concerns a method for analysing the gas of a network, in particular for determining the composition and/or quality of the gas circulating in a network and/or between networks.

### BACKGROUND ART

Currently, the evaluation of the composition and/or quality of the gas circulating in the network is carried out via a collection of a gas sample and an analysis through analysers installed directly on the field or housed in specific mobile stations, or analysing the sample in an external laboratory (not on the field). In particular, sampling is carried out through the filling of analysis vessels that must be adequately flushed out and sealed to avoid contamination of the gas to be analysed. However, the ways the gas is sampled and transported to the laboratory affect the overall accuracy of the analysis; moreover, this evaluation technique is rather complex and does not allow a frequent and low-cost analysis of the gas.

Generally, the gas chromatographic technique is used to carry out a field evaluation of gas quality. In particular, this technique requires the collection of a small aliquot of sample from the main network line and the injection into the chromatographic column for the analysis. The collected sample is consequently mixed with carrier gas to ensure its transport along the analysis apparatus and the consequent separation of the components of the gaseous mixture. Due to this mixing, the analysed gas (which is in minimal quantity with respect to the carrier gas used for the analysis) is subsequently released into the atmosphere.

Moreover, gas chromatography analysis requires a prior reduction of the pressure of the gas to be analysed. In fact, gas chromatography is not always able to operate on the gas at the pressure of the transport or distribution line since the columns and detectors currently available on the market are not designed to operate at this pressure, and moreover the pressure crucially affects the separation effects of the chromatographic column, thus reducing its effectiveness.

There are other types of analysers that do not involve mixing natural gas (or combustible gas) with a carrier gas, and these are for example inferential analysers or instruments based on optical methods of analysis. In these cases, the gas is conveyed into an analytical cell that can operate in a limited operating pressure range, generally at pressures below 4 barg. This involves the need to reduce the pressure of the gas to be analysed from the network value to the operating pressure of the cell; this pressure reduction severely limits the possibility that the analysed gas could then be re-introduced into the network and, consequently, it is released into the atmosphere. Moreover, current optical instruments for gas analysis on the field are generally quite expensive and/or inaccurate.

Basically, according to conventional techniques, the field analysis of the quality of the gas circulating in the network involves the construction of a dedicated sampling line with reduction of the gas pressure in the transport or distribution line to the operating pressure of the analyser and the collection of a small aliquot of combustible gas. Moreover, the analysis is preceded by a gas flushing phase in the sampling line pipe that brings the gas to the analyser, in order to ensure that the gas sample thus collected is representative of the mixture present in the network. The gas that is flushed into the sampling line during this procedure is then released into the surrounding environment. This involves the introduction of gas into the atmosphere, contributing, in the case of methane, to the greenhouse effect and thus going against the objective of the Paris agreements and the European Climate Law in force since July 2021 which sets out the EU's objectives of achieving climate neutrality by 2050 and reducing greenhouse gas emissions by at least 55% compared to 1990 by 2030.

US2017/314383 describes instrumentation for determining the content of a multiphase flow, such as the flow circulating in an oil pipeline.

### OBJECTS OF THE INVENTION

The object of the invention is to propose an installation and a method for the analysis of gas, which allow to overcome, at least in part, the drawbacks of the known solutions.

Another object of the invention is to propose an installation and a method that allow to analyse in an accurate, precise and reliable way the gas circulating in a network and/or between networks.

Another object of the invention is to propose an installation and a method to analyse the composition, the thermodynamic characteristics and/or the quality of the gas circulating in a network and/or between networks.

Another object of the invention is to propose an installation and a method that can be implemented in a simple and cost-effective way.

Another object of the invention is to propose an installation and a method that can be implemented in an industrial context.

Another object of the invention is to analyse continuously and on the field the gas circulating in a network and/or between networks.

Another object of the invention is to avoid the discharge of the analysed gas into the atmosphere.

Another object of the invention is to reintroduce the analysed gas into the network.

Another object of the invention is to carry out the analysis at substantially the same network pressure or in any case under high pressure conditions (e.g. up to 16 - 20 barg).

Another object of the invention is to use components already ATEX certified on the market.

Another object of the invention is to avoid the use of a gas carrier.

Another object of the invention is to propose a solution that can be used safely in potentially explosive environments.

Another object of the invention is to propose an installation and a method that can be used in different points of the production and/or distribution network of the gas mixture.

Another object of the invention is to propose an installation and a method that does not interfere with the gas transportation and/or distribution network.

Another object of the invention is to propose an installation and a method that provide analysis and measurements substantially in real time.

Another object of the invention is to propose an installation and a method that are minimally invasive and non-destructive.

Another object of the invention is to propose a method for the analysis of gas in a network that can be implemented in real time.

Another object of the invention is to propose a method for the analysis of gas in the network that is easily executable remotely.

Another object of the invention is to propose an installation and a method that are an improvement and/or alternative to traditional solutions.

Another object of the invention is to propose an installation and a method with an alternative characterization, both in functional and implementation terms, compared to the traditional ones.

### SUMMARY OF THE INVENTION

All the objects here mentioned, considered either individually or in any combination thereof, and also others which will result from the following description are attained, according to the invention, by an installation according to claim 1 and by a method according to claim 13.

### DETAILED DESCRIPTION OF THE FIGURES

The present invention is further clarified hereinafter in some of its preferred practical embodiments reported for purely exemplifying and non-limiting purposes with reference to the attached boards of drawings, wherein:
- Figure 1: shows in schematic view an installation according to the invention in a first embodiment thereof, and
- Figure 2: shows in schematic view an installation according to the invention in a second embodiment thereof.

### DETAILED DESCRIPTION OF THE INVENTION AND OF SOME PREFERRED EMBODIMENTS

The present invention concerns an installation - which, in the attached figures, is indicated globally with the reference number 1 - for the analysis of the gas circulating in a network 10 and/or between networks.

Installation 1 is configured to analyse a gas flow circulating in a network 10 and/or between networks, where said network 10 comprises a main gas circuit 11 for the passage of the gas with a first point 12 upstream and a second point 13 downstream connected to each other by a main gas line 14 for the passage of the gas. Conveniently, the network 10 can be a gas transportation or distribution network.

Preferably, only gas circulates in said network 10 and/or between the networks or in any case the presence of other phases is negligible. Conveniently, the flow to be analysed and circulating in the network 10 and/or between networks is a gas flow, and is not a multiphase mixture.

The installation 1 comprises an analysis circuit 20 with a further line 21 for the passage of the gas to be analysed.

Said further line 21:
- at the inlet and upstream is fluidically connected or connectable with the first point 12 of the main circuit 11, to thus draw/receive the gas to be analysed from the main circuit, and
- at the outlet and downstream it is fluidically connected or connectable with the second point 13 of the main circuit 11, to thus reintroduce the analysed gas into the main circuit.

In particular, the analysis circuit 20 comprises a further line 21 which is interposed between the first point 12, which corresponds to the point of collection of the gas from the main circuit 11 for its introduction into the analysis circuit, and the second point 13, which corresponds to the point of re-introduction of the gas analysed from the analysis circuit into the main circuit 11.

Essentially, the first point 12 serves as a gas collection point, for the further line 21, from the main line 14 while the second point 13 serves as a gas re-introduction point from the further line 21 into the main line 14. The first point 12 is connected to the second point 13 through the main line 14 and also through the further line 21 which, substantially, is in parallel to the main line 14.

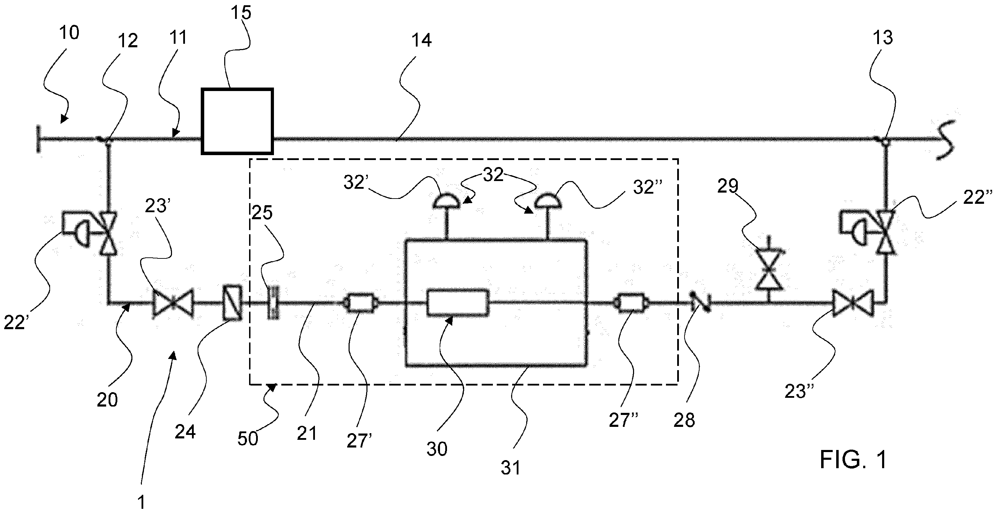

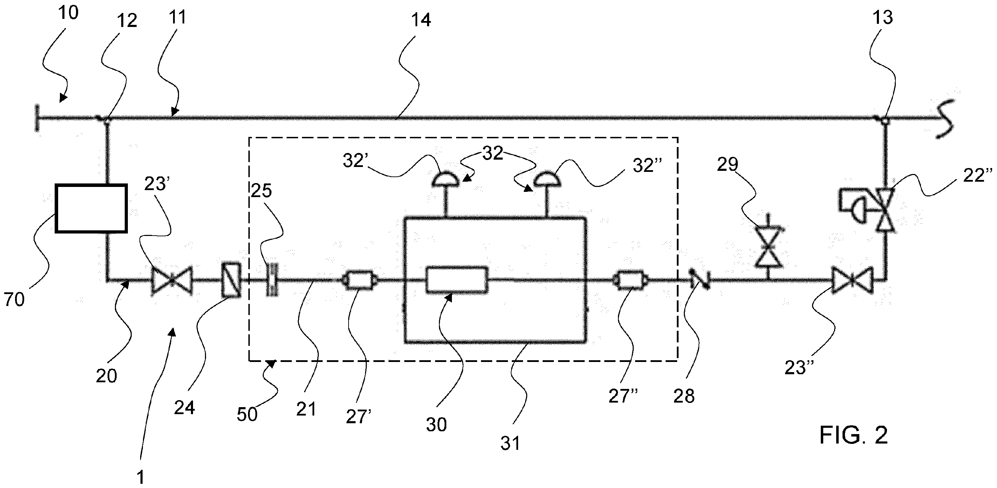

Conveniently, a gas pressure difference (i.e. a ΔP) can be generated on/along the main line 14 between the first point 12 and the second point 13 (cf. Fig. 1) and/or on/along the further line 21 between the first point 12 and the second point 13 (cf. Fig. 2). Advantageously, the difference in gas pressure (i.e. a ΔP) between the first point 12 and the second point 13 allows to reintroduce the gas entering and circulating in the further line 21 of the analysis circuit 20 into the main line 14. Preferably, a reduction in gas pressure is generated (or in any case is already provided) on/along the main line 14 between the first point 12 and the second point 13 or an increase in gas pressure is generated on/along the further line 21 between the first point 12 and the second point 13.

Conveniently, in a first possible embodiment, a reduction of the gas pressure on/along the main line 14 is provided between the first point 12, wherein the gas is at a pressure Pm, and the second point 13 wherein the gas is at a pressure Pv that is lower than Pm. Preferably, the pressure Pm of the first point 12 can be about 0.5 -100 barg; preferably, the pressure Pv of the second point 13 can be about 0 - 20 barg.

In particular, in a first possible embodiment (cf. Fig. 1), a reduction in gas pressure between the first point 12 and the second point 13 can be provided, which is generated by means of pressure reduction means 15 installed on the main line 14. More specifically, said pressure reduction means 15 may comprise at least one gas pressure regulation stage (e.g. comprising one or more pressure reducers) which is configured to reduce the pressure of the gas passing from the first point 12 to the second point 13 passing through said main line 14, or may comprise at least one control valve, a bottleneck, a curved section, or other configurations or conformations of the pipe of the main line 14 which cause a reduction in gas pressure between the first point 12 and the second point 13. Preferably, for example, the regulation stage of the pressure reduction means 15 installed on the main line 14 reduces the gas pressure from Pm, which can be about 0.5 - 100 barg, to Pv, which can be about 0 - 20 barg.

Conveniently, in a second possible embodiment, an increase in the gas pressure is provided on/along the further line 21 of the analysis circuit 20 between the first point 12, wherein the gas is at a pressure Pm, and the second point 13 wherein the gas is reintroduced into the main line 14 at a pressure Pv which is higher than Pm. In particular, in a second possible embodiment (cf. Fig. 2), on/along the further line 21 between the first point 12 and the second point 13 means 70 can be installed for increasing the pressure of the gas entering and circulating in said further line 21. More in detail, these means 70 for increasing the gas pressure may comprise a compressor, or other component, installed on the further line 21, suitable for causing an increase in the pressure of the gas which from the first point 12 enters the further line 21 and then returns to the main line 14 at the second point 13.

In particular, in the event that no means are provided along the main line 14 to reduce the gas pressure between the first point 12 and the second point 13 or in any case there is no appropriate pressure reduction between the first point 12 and the second point 13 of the main line, on/along the further line 21 between the first point 12 and the second point 13 means 70 are installed or provided for increasing the pressure of the gas entering and circulating in said further line 21.

Conveniently, in a possible embodiment, the installation 1 may comprise, in addition to the analysis circuit 20, also the main circuit 11 of a gas network and/or between gas networks.

Conveniently, in a possible embodiment, the installation 1 may comprise the analysis circuit 20 to be associated with a main circuit 11 of a gas network and/or between gas networks.

The analysis circuit 20 of the installation 1 also comprises an analyser device 30, preferably of the optical type, which is mounted on the further line 21 of the analysis circuit 20 between the first point 12 and the second point 13 and which is configured to analyse the gas passing through the further line 21 of the analysis circuit 20.

The further line 21 of the analysis circuit 20 is in parallel to the main line 14 of the main circuit 11 and, in particular, substantially defines a bypass line, with respect to the main line 14, between the first point 12 and the second point 13 of the main circuit 11.

Conveniently, between the first point 12, which is fluidically connected with the inlet of the analysis circuit 20, and the second point 13, which is fluidically connected to the outlet of the analysis circuit 20, there is therefore a difference in gas pressure, and this allows to establish a substantially continuous gas flow that passes from the first point 12 to the second point 13 passing through the analysis circuit 20, and in particular passing through the further line 21 of the analysis circuit 20; in this way, therefore, a part of the gas, which is representative of the processed gas that also passes through the main line 14 of the main circuit 11, is passed through the inside of the analyser device 30 installed on the further line 21 of the analysis circuit, ensuring a constant exchange of the gas and this is carried out without foreign gases and without variations in the state/phase of the gas, that is without any alteration of the gas to be analysed.

Advantageously, operating in a continuous flow, the same gas that passes through the main circuit 11 enters the analysis circuit 20, or in any case, as the composition of the gas in the main circuit 11 varies, the difference in the composition of the gas that passes through the analysis circuit 20 is substantially negligible.

Conveniently, the pressure difference between the first point 12 and the second point 13 can be specifically generated, for example by using an additional device (such as a pump or compressor) or static methods (such as a pitot tube, a diaphragm to create an in-line pressure drop, etc.), or an existing pressure difference between the first point 12 and the second point 13 can be exploited, for example the pressure drop present in a decompression cabin/unit.

Conveniently, the possible means 70 for increasing the gas pressure in the further line 21 can be installed upstream (as in fig. 2), or (in a possible embodiment not illustrated in the figures) the means 70 could be installed in the further line 21 downstream of the analyser device 30 (for example in the case of a network pressure of the main line 14 which is greater than 20barg, there may first be a reduction in the gas pressure to a value lower than 20 barg for the inlet into the analyser and then a subsequent increase, through the means 70, of the gas pressure to a value greater than 20 barg to thus reinject the gas into the main line 14 at the second point 13).

The gas to be analysed and circulating in the network 10 may comprise natural gas, combustible gases obtained from renewable sources or industrial processes, technical gases and mixtures thereof. In particular, the gas to be analysed and circulating in the network 10 can be a gas mixture, for example it can be a gaseous mixture containing at least one of natural gas, biomethane and natural gas mixed with hydrogen. Preferably, the gas to be analysed and circulating in the network 10 can be a gas mixture comprising natural gas, biomethane and natural gas mixed with hydrogen. Preferably, the gas to be analysed and circulating in the network 10 is a gaseous mixture containing at least one of natural gases, combustible gases obtained from renewable sources or industrial processes, technical gases.

Conveniently, the analysis circuit 20 is gas-tight and, in particular, is configured to be able to operate (withstand) at a gas pressure greater than or equal to the minimum working gas pressure in the main circuit 11. More specifically, considering that the gas pressure in the main circuit 11 is variable in the range 0.5 - 100 barg, the analysis circuit 20 is configured to operate at a gas pressure of about 0.1 - 20 barg, preferably of 0.5 - 16 barg.

The analyser device 30 is configured to determine the quality of the gas and/or one or more gas components.

The analyser device 30 can be configured to determine the composition of the gas. More preferably, the analyser device 30 is configured to determine the concentration of at least one of the chemical species contained in it. In particular, the analyser device 30 is configured to determine the main components of natural gas (methane, heavier hydrocarbons, nitrogen, carbon dioxide, hydrogen) and/or other components present in the gas mixture.

The analyser device 30 can be configured to determine the gas quality. The analyser device 30 is configured to determine at least one parameter representative of the thermodynamic characteristics/properties of the gas. Preferably, the device is configured to determine/calculate at least one representative parameter of the gas quality, in particular in terms of its thermodynamic characteristics. Preferably, said at least one representative parameter of the gas quality is the higher calorific value (also called "HCV"). Preferably, the analyser device 30 is configured to determine at least one representative parameter of the gas quality according to the standards of the field, for example the ISO 6976:2016 standard. More preferably, the analyser device 30 is configured to determine at least one of the following parameters representative of the gas quality: higher calorific value (HCV), lower calorific value (LCV), higher and lower Wobbe indices, Z-compressibility factor, density and relative density.

More preferably, in a possible embodiment, the analyser device 30 is configured to determine the composition and the higher calorific value (HCV) of a gaseous mixture of natural gas, biomethane and natural gas mixed with hydrogen.

The analyser device 30 comprises an analysis cell which is provided with an inlet and an outlet in fluidic communication with the further line 21, to allow respectively the inlet and outlet of the gas to be analysed into/from said cell of the analyser device 30. Conveniently, the analysis cell of the analyser device 30 is gas-tight.

The analysis circuit 20 of the installation 1 comprises at least one enclosure 31 for housing the analyser device 30 or to house at least the analysis cell of the analyser device 30. Preferably, at least the analysis cell of said analyser device 30 is housed inside the enclosure 31.

Conveniently, the enclosure 31 for housing the analyser device 30 is flame-proof and explosion-proof and therefore suitable for installation in potentially explosive environments. Preferably, the enclosure 31 for housing the analyser device 30 is configured to prevent that, in the event of a flame and/or explosion, the latter spread outside the enclosure itself.

Preferably, the analyser device 30 is of the optical type and does not require any foreign gas (e.g. helium, argon), thus not polluting the gas to be analysed.

Conveniently, the analysis cell of the analyser device 30 (in terms of structure, optical windows and their coupling/assembly in the structure) is suitable for withstanding an internal pressure of up to 16 - 20 barg and, in particular, it is suitable for withstanding pressurized gases and, more specifically, gases at a pressure of up to about 16 - 20 barg. Conveniently, the analysis cell of the analyser device 30 is pressurized and, in particular, is configured to be able to withstand a pressure inside the cell, which derives from the gas to be analysed, of about 0.1 - 20 barg, preferably 0.5 - 16 barg.

More preferably, the analyser device 30 is of the optical type and uses Raman spectroscopy. In a possible and preferred embodiment, the analyser device 30 is an optical instrument capable of determining the composition of the gaseous mixture by means of Raman spectroscopy and of calculating the quality properties of the gas at reference conditions using the equations described in the ISO 6976:2016 standard. For example, the analyser device 30 can be of the type described in EP3748339 or in WO2022199928.

Alternatively, the analyser device 30 can use absorption spectrometry or other traditional analyser devices that can detect parameters such as thermal and electrical conductivity, thermal diffusivity, speed of sound, viscosity, and/or density.

Conveniently, the analyser device 30 may comprise a control and power unit that collects and transmits data to a processing and communication unit, which calculates the information relating to gas quality and transmits it to other external devices.

In a possible and preferred embodiment, the installation 1 according to the invention
- and in particular the analyser device 30 - is suitable for operating in a wide range of operating conditions, and in particular in the following conditions:
- ambient temperature ranging from -20°C to +50°C
- gas temperature ranging from -20°C to +50°C
- gas pressure ranging from 0.5 barg to 16 - 20 barg.

The gas to be analysed is collected directly from a duct of the main circuit 11, at the first point 12, in order to introduce it into a duct of the analysis circuit 20. Preferably, at the first point 12, the mechanical and fluidic connection between the main circuit 11 and the analysis circuit 20 is made by means of at least one suitable fitting, e.g. by using a so-called threaded weld fitting (also called *"weldolet")* and/or a compression fitting.

Conveniently, the analysis circuit 20 of the installation 1 may comprise at least a pressure regulator 22', 22" to control the gas pressure in the analysis circuit 20.

Preferably, the analysis circuit 20 of the installation 1 may comprise a first pressure regulator 22' which is installed on the further line 21 upstream of the analyser device 30. Advantageously, the first pressure regulator 22' allows to stabilize the gas pressure inside the analysis cell of the analyser device 30 in order to be able to operate in the most appropriate analysis conditions.

Preferably, the analysis circuit 20 of the installation 1 may comprise a second pressure regulator 22" which is installed on the further line 21 downstream of the analyser device 30. Advantageously, the second pressure regulator 22" allows the gas pressure to be brought to the appropriate reinjection conditions in the main line of the main circuit 11 and, moreover, acts as a "back-pressure" regulator to maintain the gas pressure inside the analysis cell of the analyser device 30 within certain conditions.

In a possible embodiment (not shown), a flow meter may be provided upstream of the second pressure regulator 22" to ensure a flow limitation, and consequently the control of the return pressure ("back-pressure"), in particular in some possible working conditions of the network.

Preferably, the analysis circuit 20 of the installation 1 may comprise at least one isolation valve 23', 23". More preferably, the analysis circuit 20 of the installation 1 may comprise two isolation valves 23" and 23" mounted on the further line 21, respectively upstream and downstream of the analyser device 30, to thus isolate this device from the rest of line 21 if necessary. More in detail, a first isolation valve 23" can be interposed between the first regulator 22" and the analyser device 30, while a second isolation valve 23" can be interposed between the analyser device 30 and the second regulator 22".

Preferably, the analysis circuit 20 of the installation 1 may comprise at least one flow limiter (reducer) 25 which is mounted on the further line 21 upstream of the analyser device 30, and in particular is mounted externally and upstream of the enclosure 31 for housing the analyser device 30. Conveniently, the flow limiter 25 is configured to limit/reduce the maximum flow rate of the flow on the basis of the discharge means 32 mounted on the enclosure 31 for housing the analyser device 30. More preferably, the flow limiter 25 comprises an orifice. Ideally, the flow limiter 25 comprises a nozzle.

Preferably, the analysis circuit 20 of the installation 1 may comprise at least one filter 24 which is mounted on the further line 21 upstream of the flow limiter 25. More preferably, the analysis circuit 20 of the installation 1 may comprise two membrane filters (e.g. with pore sizes of about 7 µm and 0.1 µm). Advantageously, at least one filter 24 allows to avoid the risk of obstruction of the passage hole of the flow limiter 25.

Preferably, the analysis circuit 20 of the installation 1 may comprise at least a flame arrester 27" and 27" that is mounted on the further line 21. More preferably, the analysis circuit 20 of the installation 1 may comprise two flame arresters 27" and 27" mounted on the further line 21, respectively a first flame arrester 27" that is mounted upstream (ideally immediately upstream) to the enclosure 31 for housing the analyser device 30 and a second flame arrester 27" that is mounted downstream (ideally immediately downstream) with respect to the enclosure for housing the analyser device 30. Conveniently, the flow limiter 25 is mounted upstream of the first flame arrester 27' which, in turn, is upstream of the enclosure 31 for housing the analyser device 30.

Discharge means 32 are mounted on the enclosure 31 for housing the analyser device 30 to release the gas outside the enclosure in the event that the gas pressure inside the enclosure itself exceeds a certain value, in particular a predefined limit pressure value (e.g. about 100mbar). Preferably, the discharge means 32 may comprise at least one safety valve 32', 32". More preferably, one or more safety valves 32' and 32" are installed on the enclosure 31 for housing the analyser device 30 which are configured to flush out into the atmosphere the pressurised gas from the enclosure 31 in the event of failure of the analyser device 30.

Preferably, the analysis circuit 20 of the installation 1 may comprise a non-return valve 28 which is mounted on the further line 21 downstream of the analyser device 30, to avoid reversal of the flow in the event of high pressure in the second point 13 of the main line 14. More preferably, the non-return valve 28 is positioned downstream of the second flame arrester 27".

Preferably, the analysis circuit 20 of the installation 1 may comprise a vent valve 29 which is mounted on the further line 21 downstream of the analyser device 30. More preferably, said vent valve 29 is positioned upstream of the second isolation valve 23".

Preferably, the analysis circuit 20 of the installation 1 comprises at least one safety device to ensure that the gas pressure within the analysis circuit 20 remains within a predefined value. Conveniently, said at least one safety device can be of the traditional type, it can be a manual reset (blocks) or overflow device, it can be a block operating as a monitor to reduce the gas pressure downstream, or a safety block to block the flow of the gas in the downstream direction, and it can be incorporated into the first regulator 22" and/or the second regulator 22".

In particular, the analysis circuit 20 of the installation 1 comprises an assembly 50 - which in turn comprises the flow limiter 25, the enclosure 31 for housing the analyser device 30 with the discharge means 32, the flame arresters 27' and 27" positioned immediately upstream and downstream of the enclosure 31 - which is suitable to be used in potentially explosive environments (atmospheres), and more in detail meets the safety requirements established by the regulations, directives and/or certifications expected in this regard (such as for example the ATEX certification).

In more detail, for example, the assembly 50 has been evaluated according to Annex G of standard 60079-1 and considering the system as a "Limited Release Containment System" as indicated in paragraph G.3.3 (considering Figure G.1 of Annex G of standard 60079-1, the analysis cell of analyser device 30 is the "containment system"). Preferably, the assembly 50 is according to:
- ATEX EN IEC 60079-0:2018 Standard, Explosive Atmospheres - Part 0: Equipment - General requirements Edition: 7.0;
- ATEX EN 60079-1: 2014 Standard, Explosive Atmospheres - Part 1: Equipment protection by flame-proof enclosures "d" Edition: 7.0; considering in particular Annex G "Further requirements for flameproof enclosures with an internal release source (containment system)";
- IEC 60079-0: 2017 Standard, Explosive atmospheres - Part 0: Equipment - General requirements, Edition: 7.0;
- IEC 60079-1: 2014-06 Standard, Explosive Atmospheres - Part 1: Equipment protection by flameproof enclosures "d", Edition: 7.0; considering in particular Annex G "Further requirements for flameproof enclosures with an internal source of release (containment system)".

Preferably, the enclosure 31 is made of metal alloy (e.g. aluminium alloy) and can be with or without glass windows. More preferably, the enclosure 31 is in the 2GD category according to ATEX coding. Ideally, the enclosure 31 has an internal volume of about 20 - 60 litres and is configured to withstand ambient temperatures from about -60°C to about 150°C.

Preferably, the flow limiter 25 has a passage hole with a diameter of about 0.05 - 0.20 mm, more preferably about 0.08 - 0.15 mm. Preferably, the flow limiter 25 is configured to allow a maximum volume flow of approximately 50 - 200 NI/h, more preferably approximately 60 - 180 Nl/h (tested with nitrogen and 16 barg inlet pressure and atmospheric outlet pressure).

Preferably, the flame arresters 27" and 27" (also called "flame arrestors" or "flash arrestors") are of the 2GD category according to ATEX coding. Preferably, the flame arresters 27' and 27" are certified for classes IIC and/or IIB+H2 according to ATEX coding. Preferably, the flame arresters 27' and 27" allow a maximum flow of 8000 NI/h. Preferably, the flame arresters 27" and 27" are configured to withstand ambient temperatures from approximately - 66°C to approximately 70°C.

Preferably, the safety valves 32" and 32" are certified for classes IIC and/or IIB+H2 according to the ATEX coding. Preferably, the safety valves 32" and 32" are of the 2GD category according to the ATEX coding. Preferably, the safety valves 32" and 32" allow a maximum flow of 80 - 150 NI/h. Preferably, the safety valves 32" and 32" are configured to withstand ambient temperatures from about - 65°C to about 70°C.

Conveniently, all the components of the assembly 50 - and in particular the enclosure 31, the flame arresters 27' and 27" and also the safety valves 32' and 32"- are suitable to be used in potentially explosive environments (atmospheres) and, preferably, are Ex-d certified according to ATEX coding.

The configuration of the assembly 50 allows the pressure inside the enclosure 31 to be maintained between approximately 0.9 barg and 1.1 barg in the event of failure and loss of the analysis cell of the analyser device 30. In particular, in this case, the gas flow that escapes from the analysis cell inside the enclosure 31 is then completely released into the atmosphere by the discharge means 32 to thus avoid a pressure increase of more than 1.1 barg inside the enclosure 31. Preferably, the configuration of the assembly 50 allows to avoid deformation of the lamination fittings of the enclosure 31.

Conveniently, the number of safety valves 32' and 32" to be installed on the enclosure 31 is defined on the basis of the diameter of the passage hole of the flow limiter 25 and therefore the maximum volumetric flow rate of said limiter, and also on the basis of the maximum volume flow rate of each safety valve. In particular, a number of safety valves 32' and 32" are installed on the enclosure 31 such that the maximum volumetric flow rate of the safety valve(s) is equal to or equal to the maximum volumetric flow rate of the flow limiter 25.

Preferably, the enclosure 31 has a maximum volume lower than the maximum volume allowed by the safety valves 32' and 32", in particular for risk classes IIC and IIB+H2. Conveniently, therefore, the enclosure complies with the volume limit of the safety valves 32' and 32" for both risk classes IIC and IIB+H2. Conveniently, the enclosure 31 allows to obtain a number of holes on the same, for the installation of corresponding safety valves, which is greater than the number of safety valves that is necessary to evacuate the flow from inside the enclosure 31. Therefore, the structure of the enclosure 31 is configured in such a way that a sufficient number of safety valves 32' and 32" can be installed in order to ensure the evacuation of the flow from said enclosure in case of need.

Conveniently, the assembly 50 is configured so that the flow limiter 25 reduces the flow of the gas entering the analysis cell of the analyser device 30 housed in the enclosure 31 so that, in the event of damage and gas leakage by the analysis cell, the discharge means 32 mounted on the enclosure 31 are able to discharge to the outside all the gas flow thus reduced by the flow limiter 25. Advantageously, this also allows to use an enclosure 31 that withstands lower internal pressures.

In a possible and preferred embodiment, the assembly 50 is configured to be installed in potentially explosive environments and comprises:
- an enclosure 31 wherein the analyser device 30 is housed or wherein at least one analysis cell of the analyser device 30 is housed,
- a first flame arrester 27" which is mounted upstream of said enclosure 31 and a second flame arrester 27" which is mounted downstream of said enclosure 31,
- at least one flow limiter 25 which is mounted on the further line 21 upstream of the first flame arrester 27',
- discharge means 32, preferably comprising at least one safety valve 32" and/or 32", which are mounted on said enclosure 31 and which are configured to release the gas outside the enclosure in the event that the gas pressure inside the enclosure exceeds a certain value.

Preferably, the analysis circuit 20 is configured for the passage of a continuous flow of gas. Preferably, in a possible embodiment, the main circuit 11 and the analysis circuit 20 can be devoid of means - and in particular of electrovalves - for the commanded control of the flow of gas which, at the first point 12, passes from the main circuit 11 to the analysis circuit 20 and which, at the second point 13, passes from the analysis circuit 20 to the main circuit 11.

The installation 1 according to the invention allows to collect the gas in a continuous flow from the main line 14, to analyse it in the analysis circuit 20 and to reintroduced it into the main line after having analysed it, thus avoiding the release of the analysed gas into the atmosphere or into the surrounding environment.

Conveniently, the installation 1 according to the invention can be provided at a measurement point of the natural gas transportation and/or distribution network.

Preferably, the installation 1 according to the invention can be provided:
- at the control and metering cabins at the interface between the natural gas transportation and distribution network;
- at the interconnection cabins between the transport network and industrial users;
- at the measurement points upstream of the homogeneous sampling areas;
- at the introduction points of biomethane into the natural gas transportation/distribution network;
- at the points of introduction/injection of hydrogen into the natural gas transportation/distribution network;
- downstream of a mixing station;
- at points in the network wherein hydrogen and methane are separated;
- at final reduction groups;
- at utilities.

### METHOD

The present invention also concerns a method for the continuous and on the field analysis of gas circulating in a network 10 and/or between networks, wherein said network 10 and/or between networks there is provided a main gas circuit 11 for the passage of the gas with a first point 12 and a second point 13 connected to each other by a main line 14 for the passage of the gas.

The method of analysis according to the invention is characterized in that an analysis circuit 20 is used having a further line 21 for the passage of the gas to be analysed, wherein said further line 21:
- at the inlet and upstream is fluidically connected with the first point 12 of the main circuit 11, to thus receive the gas from the main circuit, and
- at the outlet and downstream it is fluidically connected or connectable with the second point 13 of the main gas circuit 11, to thus reintroduce the gas into the main circuit.
and wherein an analyser device 30 is mounted on the further line 21 of the analysis circuit 20 between the first point 12 and the second point 13, said analyser device 30 being configured to analyse the gas that passes through the further line 21 of the analysis circuit 20.

Conveniently, between the first point 12 and the second point 13 a difference in gas pressure is provided and/or generated such as to allow, at the second point 13, the reintroduction of the gas circulating in the further line 21 of the analysis circuit 20. Preferably, between the first point 12 and the second point 13 a reduction in the pressure of the gas that passes through the main circuit 11 can be provided and/or between the first point 12 and the second point 13 an increase in the pressure of the gas that passes through the further line 21 of the analysis circuit 20 can be provided.

Conveniently, the method according to the invention involves:
- continuously collecting the gas to be analysed at the first point 12 of the main circuit 11 and thus introduce it into the further line 21 of the analysis circuit 20,
- analyse the collected gas through the analyser device 30 of the analysis circuit 20,
- reintroduce the gas thus analysed into the main circuit 11 at the second point 13, and out of the analyser device 30.

Conveniently, in the method according to the invention the gas to be analysed and circulating in the network 10 and/or between networks may comprise natural gas, combustible gases obtained from renewable sources or from industrial processes and related mixtures. In particular, the gas to be analysed and circulating in the network 10 can be a gas mixture, for example it can be a gaseous mixture containing at least one of natural gas, biomethane and natural gas mixed with hydrogen. Preferably, the gas to be analysed and circulating in the network 10 can be a gas mixture comprising natural gas, biomethane and natural gas mixed with hydrogen. Preferably, the gas to be analysed and circulating in said network 10 and/or between networks is a gaseous mixture containing at least one of natural gases, combustible gases obtained from renewable sources or industrial processes, technical gases.

### NETWORK

The present invention concerns a gas transportation and/or distribution network, preferably of a gas mixture, comprising:
- a main circuit 11 for the passage of the gas with a first point 12 and a second point 13 connected to each other by a main line 14 for the passage of the gas, and
- an installation 1, as described and illustrated above, with an analysis circuit 20 comprising a further passage line 21 which at the inlet is fluidically connected with the first point 12, to thus collect the gas to be analysed, and at the outlet and downstream of the analyser device 30 is fluidically connected with the second point 13, to thus reintroduce the gas analysed by said analyser device 30 into the main circuit 11.

### ADVANTAGES

From what has been said, it is clear that the solution according to the invention is particularly advantageous because:
- it allows the composition and/or quality of the gas circulating in the network and/or between networks to be analysed on the field and continuously in a robust, reliable, accurate and precise way, in a wide range of operating conditions;
- it allows a continuous collection of the gas which is then analysed at a frequency suitable for providing a result substantially in *real time* with respect to the moment wherein the analysis is carried out, thus returning a precise picture of the composition and/or instantaneous quality of the gas in the network;
- by reinjecting the gas into the network it is thus avoided the discharge into the atmosphere;
- it does not involve the use of carriers and, therefore, does not pollute the analysed gas;
- using an analysis cell suitable for operating with pressurized gas (in particular up to about 16 - 20 barg), the analysed gas can then be reinjected into the network;
- it can be installed in potentially explosive environments;
- it can be remotely operated/executed (in particular thanks to the optical analyser device, the signal collected can be sent and analysed remotely);
- it is simple, easy and economical to produce as it uses low-cost and ATEX certified components already available on the market.

The present invention has been illustrated and described in some of its preferred embodiments, but it is understood that executive variants may be made to them in practice, without however leaving the scope of protection of this patent for industrial invention.

## Claims

1. Installation (1) for analysing a gas flow circulating in a network (10) and/or between networks, said network (10) and/or networks comprising a main circuit (11) for the passage of the gas with a first point (12) and a second point (13) connected to each other by a main line (14) for the passage of the gas, said installation (1) being **characterised in that** it comprises an analysis circuit (20) with a further line (21) for the passage of the gas to be analysed, and wherein said further line (21):
- at the inlet and upstream is fluidically connected or connectable with the first point (12), to thus collect the gas to be analysed from the main circuit (11), and
- at the outlet and downstream it is fluidically connected or connectable with the second point (13), to thus reintroduce the gas into the main circuit (11),
and **characterised in that** the analysis circuit (20) of the installation (1) also comprises an analyser device (30), preferably of the optical type, which is mounted on the further line (21) of the analysis circuit (20) between the first point (12) and the second point (13) and which is configured to analyse the gas that passes through the further line (21) of the analysis circuit (20).

2. Installation according to claim 1, wherein means (15, 70) are provided to create a difference in gas pressure between the first point (12) and the second point (13).

3. Installation according to one or more of the preceding claims, wherein means (15) are provided along said main line (14) to reduce the gas pressure between the first point (12) and the second point (13) and/or where means (70) are provided along the further line (21) to increase the gas pressure between the first point (12) and the second point (13).

4. Installation according to one or more of the preceding claims, **characterized in that**:
- said analysis circuit (20) is gas-tight, and
- it is configured to be able to operate at and withstand a gas pressure that is greater than or equal to the minimum working pressure of the gas in the main circuit (11), in particular at the first point (12), preferably it is configured to operate at an internal pressure up to about 16 - 20 barg.

5. Installation according to one or more of the preceding claims, **characterised in that**, in the event that no means are provided on the main line (14) to reduce the gas pressure between the first point (12) and the second point (13), means (70) are installed on the further line (21) between the first point (12) and the second point (13) to increase the pressure of the gas entering and circulating **in that** further line (21).

6. Installation according to one or more of the preceding claims, **characterized in that** said analyser device (30) is configured to determine the composition of the gas, preferably said analyser device (30) is optical and uses Raman spectroscopy.

7. Installation according to one or more of the preceding claims, **characterised in that** the analyser device (30) is configured to determine the quality of the gas and, preferably, to determine at least one parameter representative of the thermodynamic characteristics of the gas, such as the higher calorific value (HCV).

8. Installation according to one or more of the preceding claims, **characterized in that** said analyser device (30) comprises an analysis cell which at the inlet and outlet is in fluidic connection with the further line (21), said analysis cell is gas-tight and is configured to withstand and operate at an internal pressure up to about 16 - 20 barg.

9. Installation according to one or more of the preceding claims, **characterized in that** the analysis circuit (20) comprises an enclosure (31) wherein said analyser device (30) is housed or wherein at least one analysis cell of the analyser device (30) is housed.

10. Installation according to one or more of the preceding claims, **characterized in that** the analysis circuit (20) comprises:
- at least one pressure regulator (22', 22") that is mounted on the further line (21) upstream and/or downstream of the analyser device (30), and/or
- at least one flow limiter (25) which is mounted on the further line (21) upstream of an enclosure (31) wherein the analyser device (30) is housed.

11. Installation according to one or more of the preceding claims, **characterized in that** the analysis circuit (20) comprises a first flame arrester (27") which is mounted upstream of an enclosure (31) wherein the analyser device (30) is housed, and a second flame arrester (27") which is mounted downstream of the enclosure (31) housing the analyser device (30).

12. Installation according to one or more of the preceding claims, **characterized in that** the analyser device (30) comprises an analysis cell which is housed inside an enclosure (31) and **in that** discharge means (32) are mounted on said enclosure (32), preferably at least a safety valve (32', 32"), to release the gas outside the enclosure in the event that the gas pressure inside the enclosure exceeds a certain value.

13. Method for the continuous and on the field analysis of the gas circulating in a network (10) and/or between networks, said network (10) and/or networks comprising a main circuit (11) for the passage of the gas with a first point (12) and a second point (13) connected to each other by a main line (14) for the passage of the gas, **characterized in that** an analysis circuit (20) with a further line (21) for the passage of the gas to be analysed is used, and wherein said further line (21):
- at the inlet and upstream is fluidically connected or connectable with the first point (12) of the main circuit (11), to thus collect the gas to be analysed from the main circuit (11), and
- at the outlet and downstream is fluidically connected or connectable with the second point (13) of the main circuit (11), to thus reintroduce the gas into the main circuit (11),
and wherein:
- the analysis circuit (20) also comprises an analyser device (30), preferably of the optical type, which is mounted on the further line (21) of the analysis circuit (20) between the first point (12) and the second point (13) and which is configured to analyse the gas that passes through the further line (21) of the analysis circuit (20).

14. Method according to the previous claim, **characterized in that**:
- the gas to be analysed is continuously collected at the first point (12) to thus introduce it into the further line (21) of the analysis circuit (20),
- the gas thus collected is analysed by means of the analyser device (30) of the analysis circuit (20),
- the gas thus analysed that leaves the analyser device (30) is reintroduced into the main circuit (11) at the second point (13),
and wherein a difference in gas pressure is provided or is generated between the first point (12) and the second point (13) to thus cause, at the second point (13), the reintroduction of gas from the further line (21) into the main line (14).

15. Method according to the previous claim, **characterised in that** said gas to be analysed and circulating in said network (10) and/or between networks is a mixture of gases, preferably it is a gaseous mixture containing at least one of natural gases, combustible gases obtained from renewable sources or industrial processes, technical gases.
